(19) Europäisches Patentamt
European Patent Office
Office européen des brevets

(11) Veröffentlichungsnummer: **0 113 311 B1**

(12) # EUROPÄISCHE PATENTSCHRIFT

(45) Veröffentlichungstag der Patentschrift: **02.01.92**

(51) Int. Cl.$^5$: **C07C 405/00**, A61K 31/557

(21) Anmeldenummer: **83730094.6**

(22) Anmeldetag: **04.10.83**

(54) **5-Fluorcarbacycline, Verfahren zu ihrer Herstellung und ihre Verwendung als Arzneimittel.**

(30) Priorität: **05.10.82 DE 3237200**

(43) Veröffentlichungstag der Anmeldung:
**11.07.84 Patentblatt 84/28**

(45) Bekanntmachung des Hinweises auf die Patenterteilung:
**02.01.92 Patentblatt 92/01**

(84) Benannte Vertragsstaaten:
**AT BE CH DE FR GB IT LI LU NL SE**

(56) Entgegenhaltungen:
**GB-A- 2 070 596**

(73) Patentinhaber: **SCHERING AKTIENGESELLSCHAFT Berlin und Bergkamen**
**Müllerstrasse 170/178 Postfach 65 03 11**
**W-1000 Berlin 65(DE)**

(72) Erfinder: **Skuballa, Werner, Dr.**
**Olwenstrasse 23**
**W-1000 Berlin 28(DE)**
Erfinder: **Radüchel, Bernd, Dr.**
**Gollanczstrasse 132**
**W-1000 Berlin 28(DE)**
Erfinder: **Vorbrüggen, Helmut, Prof. Dr.**
**Wilkestrasse 7**
**W-1000 Berlin 27(DE)**
Erfinder: **Haberey, Martin Dr.**
**Neckarsulmer Strasse 15**
**W-1000 Berlin 46(DE)**
Erfinder: **Stürzebecher, Claus-Steffen**
**Kuckucksweg 6**
**W-1000 Berlin 33(DE)**
Erfinder: **Town, Michael-Harold, Dr.**
**Zugspitzstrasse 13**
**W-8127 Iffeldorf(DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Rank Xerox (UK) Business Services

## Beschreibung

Die Erfindung betrifft neue 5-Fluorcarbacyclin-Derivate, Verfahren zu ihrer Herstellung sowie ihre Verwendung als Arzneimittel. In den deutschen Offenlegungsschriften DE-OS 28 45 770, 29 00 352, 29 02 442, 20 04 655, 29 09 088, 30 48 906 und 29 12 409 werden (5E)- und (5Z)-6a-Carbaprostaglandin-$I_2$-Analoga beschrieben. In GB 20 70 536 werden 5-Fluor-carbacyclin-Derivate beansprucht, die jedoch keine Dreifachbindung in 14-, 18- oder 19-Stellung besitzen. Die Nomenklatur der erfindungsgemäßen Verbindungen basiert auf einem Vorschlag von Morton und Brokaw (J.Org.Chem. 44, 2280 [1979]. Bei der Synthese dieser Verbindung entstehen stets zwei Doppelbindungsisomere, die durch den Zusatz (5E) oder (5Z) charakterisiert werden. Die beiden Isomeren dieses Prototyps werden durch folgende Strukturformel verdeutlicht:

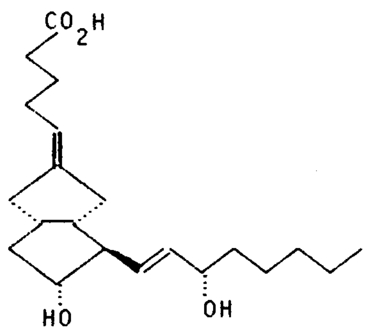

(5E)-6a-Carbaprostaglandin-$I_2$

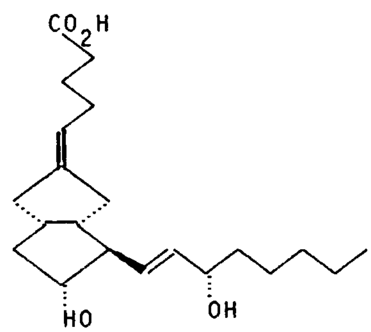

(5Z)-6a-Carbaprostaglandin-$I_2$

Aus dem sehr umfangreichen Stand der Technik der Prostacycline und ihrer Analoga weiß man, daß diese Stoffklasse aufgrund ihrer biologischen und pharmakologischen Eigenschaften zur Behandlung von Säugetieren, einschließlich Menschen geeignet ist. Ihre Verwendung als Arzneimittel stößt jedoch häufig auf Schwierigkeiten, da sie eine für therapeutische Zwecke zu kurze Wirkungsdauer besitzen. Alle Strukturveränderungen haben das Ziel, die Wirkungsdauer sowie die Selektivität der Wirksamkeit zu steigern.

Es wurde nun gefunden, daß durch Substitution des Wasserstoffatoms in der 5-Position der Carbacycline durch ein Fluoratom eine längere Wirkungsdauer, eine größere Selektivität und eine bessere Wirksamkeit erzielt werden kann. Die erfindungsgemäßen Verbindungen wirken blutdrucksenkend und bronchodilatorisch. Sie sind außerdem zur Vasodilatation, Inhibierung der Thrombocytenaggregation sowie der Magensäuresekretion geeignet.

Die Erfindung betrifft 5-Fluorcarbacyclinderivate der allgemeinen Formel I

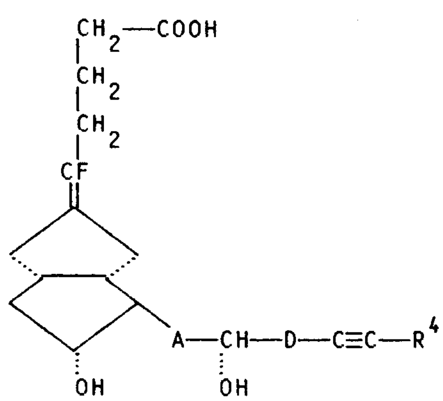

(I),

worin A eine trans-CH = CH- oder -C≡C-Gruppe,

D die Gruppe

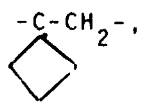

wenn $R_4$ Ethyl bedeutet, oder die Reste $-CH(CH_3)-CH_2-$ oder $-C(CH_3)_2-CH_2-$,

$R^4$ Methyl oder Ethyl bedeuten und deren Salze mit physiologisch verträglichen Basen.

Die Verbindungen der Formel I stellen sowohl (5E)- als auch (5Z)-Isomere dar.

Zur Salzbildung mit den freien Säuren sind anorganische und organische Basen geeignet, wie sie dem Fachmann zur Bildung physiologisch verträglicher Salze bekannt sind. Beispielsweise seien genannt: Alkalihydroxide, wie Natrium- und Kaliumhydroxid, Erdalkalihydroxide, wie Calciumhydroxid, Ammoniak, Amine, wie Ethanolamin, Diethanolamin, Triethanolamin, N-Methylglucamin, Morpholin, Tris-(hydroxymethyl)-methylamin usw.

Die Erfindung betrifft ferner ein Verfahren zur Herstellung der erfindungsgemäßen 5-Fluorcarbacyclin-Derivate der allgemeinen Formel I, dadurch gekennzeichnet, daß man in an sich bekannter Weise eine Verbindung der allgemeinen Formel II

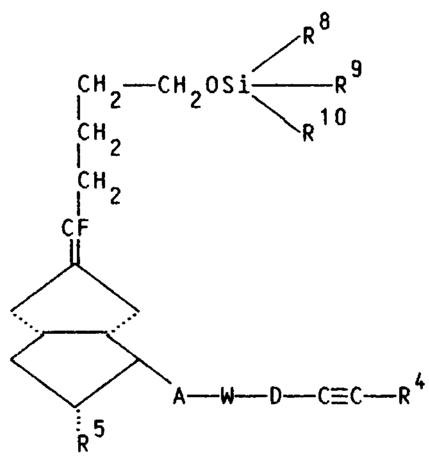

(II),

worin $R^4$, A und D die oben angegebenen Bedeutungen aufweisen, $R^5$ eine freie oder geschützte Hydroxygruppe, W eine freie oder geschützte Hydroxymethylengruppe und $R^8$, $R^9$ und $R^{10}$ gleich oder verschieden sein können und eine geradkettige oder verzweigtkettige Alkylgruppe mit 1-10 C-Atomen oder eine Arylgruppe bedeuten, gegebenenfalls nach Schutz anwesender freier Hydroxygruppen mit einem Reagenz, das zur Spaltung von Silylgruppen geeignet ist, umsetzt und gegebenenfalls anschließend in beliebiger Reihenfolge Isomere trennt und/oder geschützte Hydroxygruppen freisetzt und die 1-Hydroxygruppe zur 1-Carboxylgruppe oxidiert und geschützte Hydroxygruppen freisetzt oder mit einer physiologisch verträglichen Base in ein Salz überführt.

Die Abspaltung der Silylethergruppen in den Verbindungen der allgemeinen Formel II erfolgt mit einem Tetraalkylammoniumfluorid, vorzugsweise Tetrabutylammoniumfluorid, oder einem Alkali- oder Erdalkalifluorid in einem inerten Lösungsmittel wie beispielsweise Tetrahydrofuran, Diethylether, Dioxan, Dimethoxyethan, Methylenchlorid, Dimethylformamid, Dimethylsulfoxid usw. bei Temperaturen zwischen 0° und 80° C, vorzugsweise 20° bis 45° C.

Die Oxidation der 1-Hydroxygruppe wird nach den dem Fachmann bekannten Methoden vorgenommen. Als Oxidationsmittel können beispielsweise dienen: Pyridiniumdichromat (Tetrahedron Letters, 1979, 399), Jones-Reagenz (J. Chem. Soc. 1953, 2555) oder Platin/Sauerstoff (Adv. in Carbohydrate Chem. 17, 169 (1962) oder Collins-Oxidation und anschließende Jones-Oxidation. Die Oxidation mit Pyridiniumchromat wird bei Temperaturen von 0° C bis 100° C, vorzugsweise 20° C bis 40° C in einem gegen das Oxidationsmittel inerten Lösungsmittel, beispielsweise Dimethylformamid, durchgeführt.

Die Oxidation mit Jones-Reagenz wird bei Temperaturen von -40° C bis +40° C, vorzugsweise 0° C bis 30° C in Aceton als Lösungsmittel ausgeführt.

Die Oxidation mit Platin/Sauerstoff wird bei Temperaturen von 0° C bis 60° C, vorzugsweise 20° C bis 40° C in einem gegen das Oxidationsmittel inerten Lösungsmittel, wie z. B. Essigester, durchgeführt.

Die Carbacylinderivate der allgemeinen Formel I können mit geeigneten Mengen der entsprechenden

anorganischen Basen unter Neutralisierung in Salze überführt werden. Beispielsweise erhält man beim Lösen der entsprechenden Säuren in Wasser, welches die stöchiometrische Menge der Base enthält, nach Abdampfen des Wassers oder nach Zugabe eines mit Wasser mischbaren Lösungsmittels, zum Beispiel Alkohol oder Aceton, das feste anorganische Salz.

Die Herstellung der Aminsalze erfolgt in üblicher Weise. Dazu wird die Carbacyclinsäure zum Beispiel in einem geeigneten Lösungsmittel wie Äthanol, Aceton, Diäthyläther oder Benzol, gelöst und mindestens die stöchiometrische Menge des Amins dieser Lösung zugesetzt. Dabei fällt das Salz gewöhnlich in fester Form an oder wird nach Verdampfen des Lösungsmittels in üblicher Weise isoliert.

Der Schutz der freien OH-Gruppen erfolgt nach den dem Fachmann bekannten Methoden. Zur Einführung der Ätherschutzgruppen wird beispielsweise mit Dihydropyran in Methylenchlorid oder Chloroform unter Verwendung eines sauren Kondensationsmittels, wie zum Beispiel p-Toluol-sulfonsäure, umgesetzt. Das Dihydropyran wird im Überschuß angewandt, vorzugsweise in der 4- bis 10fachen Menge des theoretischen Bedarfs. Die Umsetzung ist normalerweise bei 0° C - 30° C nach 15 bis 30 Minuten beendet.

Die Einführung der Acylschutzgruppen erfolgt, indem man eine Verbindung der allgemeinen Formel I in an sich bekannter Weise mit einem Carbonsäurederivat, wie zum Beispiel Säurechlorid, Säureanhydrid u.a., umsetzt.

Die Freisetzung einer geschützten OH-Gruppe zu den Verbindungen der allgemeinen Formel I erfolgt nach bekannten Methoden. Beispielsweise wird die Abspaltung von Ätherschutzgruppen in einer wäßrigen Lösung einer organischen Säure, wie zum Beispiel Essigsäure, Propionsäure u.a. oder in einer wäßrigen Lösung einer anorganischen Säure, wie zum Beispiel Salzsäure, durchgeführt. Zur Verbesserung der Löslichkeit wird zweckmässigerweise ein mit Wasser mischbares inertes organisches Lösungsmittel zugesetzt. Geeignete organische Lösungsmittel sind zum Beispiel Alkohole, wie Methanol und Äthanol, und Äther, wie Dimethoxyäthan, Dioxan und Tetrahydrofuran. Tetrahydrofuran wird bevorzugt angewendet. Die Abspaltung wird vorzugsweise bei Temperaturen zwischen 20° C und 80° C durchgeführt.

Die Abspaltung der Silylätherschutzgruppen erfolgt beispielsweise mit Tetrabutylammoniumfluorid. Als Lösungsmittel sind beispielsweise geeignet Tetrahydrofuran, Diäthyläther, Dioxan, Methylenchlorid usw. Die Abspaltung wird vorzugsweise bei Temperaturen zwischen 0° C und 80° C durchgeführt.

Da die Endprodukte freie Hydroxylgruppen im Prostanrest enthalten, geht man zweckmäßigerweise von Ausgangsprodukten aus, in denen diese durch vorzugsweise leicht abspaltbare Ether- oder Acylreste intermediär geschützt sind.

Die als Ausgangsmatertial dienenden Verbindungen der allgemeinen Formel II können beispielsweise hergestellt werden, indem man in an sich bekannter Weise einen Aldehyd der Formel IV (DE-OS 28 45 770)

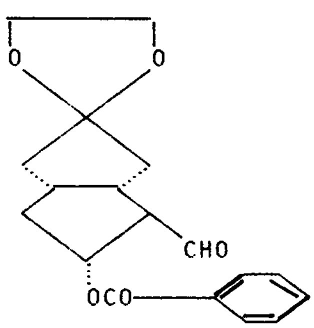

(IV)

mit einem Phosphonat der allgemeinen Formel V

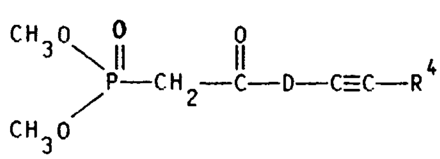

(V)

worin D und $R^4$ die oben angegebene Bedeutung aufweisen, in Gegenwart eines Deprotonierungsmittels,

wie beispielsweise Natriumhydrid oder Kalium-tert.-butylat, zu einem Keton der allgemeinen Formel VI (X = H) oder zusätzlich in Gegenwart eines Bromierungsmittels, wie beispielsweise N-Bromsuccinimid, zu einem Keton der allgemeinen Formel VI (X = Br) umsetzt.

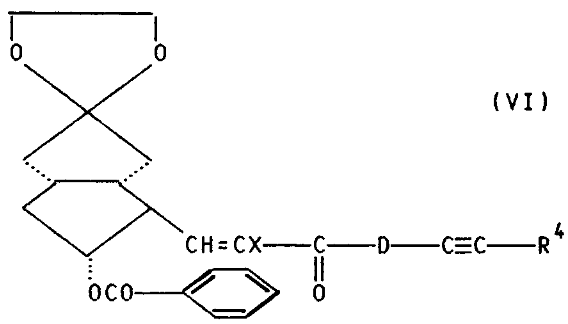

(VI)

Nach Reduktion der Ketogruppe mit Zinkborhydrid oder Natriumborhydrid oder Umsetzung mit Alkylmagnesiumbromid oder Alkyllithium und anschließender Epimerentrennung gelangt man zu den Verbindungen der allgemeinen Formel VII

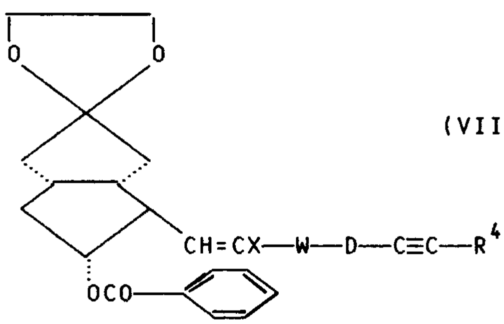

(VII)

Verseifung der Estergruppe, beispielsweise mit Kaliumcarbonat in Methanol, sowie gegebenenfalls Hydrierung der Doppelbindung oder gegebenenfalls Veretherung mit Dihydropyran und anschließende Abspaltung von Bromwasserstoff mit beispielsweise Kalium-tert.-butylat in Dimethylsulfoxid, Ketalspaltung mit wäßriger Essigsäure, gegebenenfalls funktionelle Abwandlung der freien Hydroxygruppen, beispielsweise durch Veretherung mit Dihydropyran liefert das Keton der allgemeinen Formel VIII

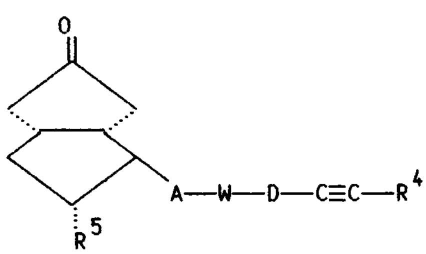

(VIII)

Die Umsetzung des Ketons VIII mit einem aus dem Ester der allgemeinen Formel IX (worin

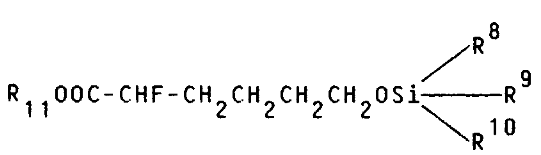

(IX)

$R^8$, $R^9$, $R^{10}$ die oben angegebenen Bedeutungen aufweisen und $R^{11}$ eine Alkylgruppe mit 1 bis 5 C-Atomen

bedeutet) und Lithiumdiisopropylamid hergestellten Carbanions liefert nach Verseifung mit beispielsweise alkoholischer Kalilauge die Säure der allgemeinen Formel X

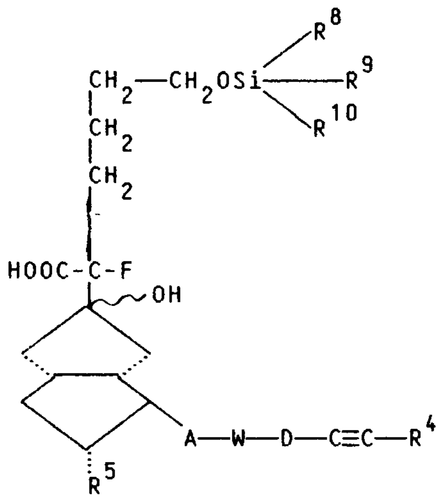

(X)

Die dehydratisierende Decarboxylierung der Hydroxycarbonsäure der allgemeinen Formel X mit Dimethylformamidacetaten oder mit Arylsulfonsäurechlorid und Pyridin und anschließender thermischer Behandlung des intermediären ß-Lactons liefert das 5-Fluorolefin der allgemeinen Formel II.

Die Herstellung der Phosphonate der allgemeinen Formel V erfolgt in an sich bekannter Weise durch Umsetzung des Anions von Methylphosphonsäuredimethylester mit einem Ester der allgemeinen Formel XI

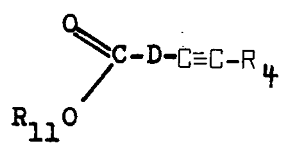

XI

worin D, E, $R_4$ die obengenannten Bedeutungen aufweisen und $R_{11}$ eine Alkylgruppe mit 1-5 C-Atomen bedeutet, den man gegebenenfalls aus dem entsprechenden Malonsäureester durch Alkylierung mit dem Halogenid

$Hal\text{-}C{\equiv}C\text{-}R_4$ XII

der allgemeinen Formel XII mit Hal in der Bedeutung Chlor oder Brom und anschließender Decarbalkoxylierung erhalten kann. Der Ester der allgemeinen Formel XI ist gegebenenfalls auch aus der Carbonsäure der allgemeinen Formel XIII

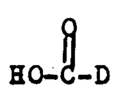

XIII

worin D die obenangegebene Bedeutung aufweist durch Alkylierung mit dem Halogenid der allgemeinen Formel XII und anschließender Veresterung zugänglich.

Die Herstellung der Fluoresters der allgemeinen Formel IX erfolgt in an sich bekannter Weise durch selektive Silylierung von 1,4-Butandiol und anschließender Oxidation mit beispielsweise Collinsreagenz oder Pyridiniumdichromat zum Aldehyd XIV.

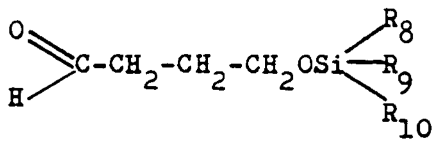

XIV

Nach Olefinierungsreaktion mit Phosphonofluoressigsäuretriäthylester und anschließender katalytischer Hydrierung erhält man den Ester der allgemeinen Formel IX.

Die Verbindungen dieser Erfindung wirken blutdrucksenkend und bronchodilatorisch. Sie sind weiterhin geeignet zur Hemmung der Thrombozyten-Aggregation. Folglich stellen die neuen Carbacyclin-Derivate der Formel I wertvolle pharmazeutische Wirkstoffe dar. Darüber hinaus weisen sie bei ähnlichem Wirkungsspektrum, verglichen mit entsprechenden Prostaglandinen, eine höhere Spezifität und vor allem eine wesentlich längere Wirksamkeit auf. Im Vergleich zu $PGI_2$ zeichnen sie sich durch größere Stabilität aus. Die hohe Gewebsspezifität der neuen Prostaglandine zeigt sich bei der Untersuchung an glattmuskulären Organen, wie zum Beispiel am Meerschweinchenileum oder an der isolierten Kaninchentrachea, wo eine wesentlich geringere Stimulation zu beobachten ist als bei der Applikation natürlicher Prostaglandine vom E-, A- oder F-Typ.

Die neuen Carbacyclin-Analoga besitzen die für Prostacycline typischen Eigenschaften, wie zum Beispiel Senkung des peripheren arteriellen und koronaren vaskulären Widerstandes, Inhibierung der Thrombozytenaggregation und Auflösung von Plättchenthromben, myocardiale Zytoprotektion und damit Senkung des systemischen Blutdruckes,ohne zugleich Schlagvolumen und koronare Durchblutung zu senken; Behandlung von Schlaganfall, Prophylaxe und Therapie koronarer Herzerkrankungen, koronarer Thrombosen, des Herzinfarktes, peripherer Arterienerkrankungen, Arteriosklerose und Thrombose, Prophylaxe und Therapie ischaemischer Attacken des ZNS-Systems, Therapie des Schocks, Inhibierung der Bronchokonstriktion, Inhibierung der Magensäuresekretion, Zytoprotektion der Magen- und Darmschleimhaut, Zytoprotektion in der Leber und im Pankreas, antiallergische Eigenschaften, Senkung des pulmonaren vaskulären Widerstandes und des pulmonaren Blutdrucks, Förderung der Nierendurchblutung, Anwendung anstelle von Heparin oder als Adjuvans bei der Dialyse der Hämofiltration, Konservierung von Blutplasmakonserven, besonders von Blutplättchenkonserven, Inhibierung von Geburtswehen, Behandlung von Schwangerschaftstoxikose, Erhöhung der zerebralen Durchblutung etc. Außerdem besitzen die neuen Carbacyclin-Derivate antiproliferative und antidiarrhoegene Eigenschaften. Die Carbacycline dieser Erfindung können auch in Kombination, zum Beispiel mit $\beta$-Blockern, Diuretika oder Phosphondiesterasehemmern verwendet werden.

Die Dosis der Verbindungen ist 1-1500 $\mu$g/kg/Tag, wenn sie am menschlichen Patienten verabreicht werden. Die Einheitsdosis für den pharmazeutischen akzeptablen Träger beträgt 0,01 - 100 mg.

Bei intravenöser Injektion an wachen, hypertonen Ratten in Dosen von 5, 20 und 100 $\mu$g/kg Körpergewicht zeigen die erfindungsgemäßen Verbindungen eine stärkere blutdrucksenkende und länger anhaltende Wirkung als $PGE_2$ und $PGA_2$, ohne wie $PGE_2$ Durchfälle und $PGA_2$ kardiale Arrhythmien auszulösen.

Bei intravenöser Injektion an narkotisierten Kaninchen zeigen die erfindungsgemäßen Verbindungen im Vergleich zu $PGE_2$ und $PGA_2$ eine stärkere und erheblich länger anhaltende Blutdrucksenkung, ohne daß andere glattmuskuläre Organe oder Organfunktionen beeinflußt werden.

Für die parenterale Verabreichung werden sterile, injizierbare, wäßrige oder ölige Lösungen benutzt. Für die orals Applikation sind beispielsweise Tabletten, Dragees oder Kapseln geeignet.

Die Erfindung betrifft damit auch Arzneimittel auf Basis der Verbindungen der allgemeinen Formel I und üblicher Hilfs- und Trägerstoffe.

Die erfindungsgemäßen Wirkstoffe sollen in Verbindung mit den in der Galenik bekannten und üblichen Hilfsstoffen, zum Beispiel zur Herstellung von Blutdrucksenkern, dienen.

## Beispiel 1

### (5Z)-(16RS)-5-Fluor-16-methyl-18,18,19,19-tetradehydro-6a-carbaprostaglandin-$I_2$

Zu einer Lösung von 960 mg (5E,Z)-(16RS-2-Descarboxy-5-fluor-16-methyl-2-(dimethyl-tert.-butyl-silyloxymethyl)-18,18,19,19-tetradehydro-6a-carba-prostaglandin-$I_2$-11,15-bis-(tetrahydropyranyläther) in 30 ml Tetrahydrofuran fügt man 0,93 g Tetrabutylammoniumfluorid und rührt 6 Stunden bei 25° C. Anschließend verdünnt man mit Äther, schüttelt dreimal mit Wasser, trocknet über Magnesiumsulfat und dampft im Vakuum ein. Den Rückstand chromatographiert man mit Hexan/Äther (3+2) an Kieselgel. Dabei erhält man als unpolarere Komponente 360 mg (5E)-(16RS-2-Descarboxy-6-fluor-2-hydroxymethyl-16-methyl-18,18,19,19-tetradehydro-6a-carba-prostaglandin-$I_2$-11,15-bis-(tetrahydropyranyläther) und als polarere Komponente 380 mg (5Z)-(16RS)-2-Descarboxy-5-fluor-2-hydroxymethyl-16-methyl-18,18,19,19-tetradehydro-6a-carba-prostaglandin-$I_2$-11,15-bis-(tetrahydropyranyläther).
IR ($CHCl_3$): 3650, 2930, 2865, 1600, 972/cm.

Zur Oxidation der 1-Hydroxygruppe löst min 380 mg des 5Z konfigurierten Fluorolefins in 10 ml Methylenchlorid, versetzt bei 0° C mit 1,8 g Collins-Reagenz (Chromsäure-pyridin-Komplexe)und rührt 15 Minuten bei 0° C. Anschließend versetzt man mit einer Mischung aus Äther/Hexan (3+2), filtriert, wäscht das Filtrat nacheinander mit Wasser, 10%ige Schwefelsäure und Wasser, trocknet über Magnesiumsulfat und dampft im Vakuum ein. Man löst anschließend den erhaltenen Aldehyd in 16 ml Aceton und versetzt bei -20° C tropfenweise mit 1,6 ml Jones-Reagenz. Man rührt 30 Minuten bei -20° C, fügt 2 ml Isopropylalkohol zu, verdünnt mit Äther, schüttelt dreimal mit Wasser, trocknet über Magnesiumsulfat und dampft im Vakuum ein.

Zur Abspaltung der Schutzgruppen rührt man den Eindampfrückstand 16 Stunden bei 25° C mit 30 ml Essigsäure/Wasser/Tetrahydrofuran (65/35/10). Man dampft unter Zusatz von Toluol ein und chromatographiert den Rückstand an Kieselgel mit Essigester/0,1-1% Essigsäure und erhält 245 mg der Titelverbindung als farblosen Öl.
IR: 3600, 3400 (breit), 2920, 2870, 1719, 1602, 1430, 969/cm.

Das Ausgangsmaterial für die obige Titelverbindung wird wie folgt hergestellt:

1a)

(5E,Z)-(16RS)-2-Descarboxy-5-fluor-16-methyl-2-(dimethyl-tert.-butylsilyloxymethyl)-18,18,19,19-tetradehydro-6a-carba-prostaglandin-$I_2$-11,15-bis-(tetrahydropyranyläther)

Eine Lösung aus 2,8 ml Diisopropylamin in 10 ml Tetrahydrofuran versetzt man bei -25° C innerhalb von 15 Minuten mit 12,3 ml einer 1,62 molaren Lösung von Butyllithium in Hexan und rührt 1 Stunde bei -25° C. In diese Mischung tropft man bei -70° C innerhalb von 10 Minuten eine Lösung aus 5,8 g 2-Fluor-6-(dimethyl-tert.-butylsilyloxy)-hexansäureäthylester in 5 ml Tetrahydrofuran. Man rührt 10 Minuten bei -70° C, fügt eine Lösung von 2,2 g (IR,5S,6R, 7R)-7-(Tetrahydropyran-2-yloxy)-6-[(E)-(3S,4RS)-4-methyl-3-(tetrahydropyran-2-yloxy)-oct-1-en-6-inyl]-bicyclo[3.3.0]octan-3-on in 15 ml Diäthyläther und 15 ml Tetrahydrofuran zu, rührt 1 Stunde bei -70° C, versetzt mit 100 ml gesätt. Ammoniumchloridlösung und säuert mit 10%iger Zitronensäurelösung auf pH5 an. Man extrahiert mit Äther, wäscht die organische Phase mit Wasser neutral, trocknet über Magnesiumsulfat und dampft im Vakuum ein.Nach Chromatographie an Kieselgel erhält man mit Hexan/Äther 1,8 g des $\alpha$-Fluoresters, den man zur Verseifung 7 Stunden mit 25 ml einer äthanolischen Kaliumhydroxidlösung (Herstellung: Man löst 5 g Kaliumhydroxid in 187 ml Äthanol und 62 ml Wasser) bei 25° C rührt. Man säuert mit 10%iger Zitronensäurelösung auf pH4 an, extrahiert mit Methylenchlorid, wäscht den organischen Extrakt mit Wasser neutral und trocknet über Magnesiumsulfat.

Den Eindampfrückstand löst man in 35 ml Pyridin, fügt bei 0° C 880 mg Benzolsulfonsäurechlorid zu und rührt 24 Stunden bei 0° C. Anschließend versetzt man mit 1,5 ml Wasser, rührt 1 Stunde,verdünnt mit Äther, schüttelt mit gesätt. Natriumbicarbonatlösung, wäscht dreimal mit Wasser, trocknet über Magnesiumsulfat und dampft im Vakuum ein. Den Rückstand rührt man 3 Stunden bei 100° C in 20 ml Pyridin und dampft unter Zusatz von Toluol im Vakuum ein. Den Rückstand chromatographiert man mit Hexan/Äther (9+1) an Kieselgel. Dabei erhält man 1,1 g der Titelverbindung als farbloses Öl.
IR: 2945, 2868, 1450, 971, 862, 835/cm.

1 b)

Herstellung von 2-Fluor-6-(dimethyl-tert.-butylsilyloxy)-hexansäureäthylester

Zu einer Lösung von 27 g 1,4-Butandiol in 50 ml Dimethylformamid fügt man bei 0° C 51 g Imidazol und 45 g Dimethyl-tert.-butylsilylchlorid und rührt 24 Stunden bei 0° C. Anschließend gießt man auf 400 ml Wasser, extrahiert mit einer Mischung aus Äther/Pentan (1+1), wäscht die organische Phase nacheinander mit 5%iger Schwefelsäure, 5%iger Natriumbicarbonatlösung und Wasser. Man trocknet über Magnesiumsulfat und dampft im Vakuum ein. Nach Filtration über Kieselgel mit Hexan/Äther (1+1) erhält man 18 g 1-(Dimethyl-tert.-butylsilyloxy)-butan-4-ol als farblose Flüssigkeit.

Zu einer Lösung von 12 g der vorstehend hergestellten Monosilyläthers in 600 ml Methylenchlorid fügt man bei 0° C 90 g Collinsreagenz und rührt 30 Minuten bei 0° C. Anschließend verdünnt man mit Äther und schüttelt nacheinander mit 5%iger Natriumbicarbonatlösung, Wasser, 10%ige Zitronensäurelösung, 5%ige Natriumbicarbonatlösung und Wasser. Man trocknet über Magnesiumsulfat und dampft im Vakuum ein.

Zu einer Suspension von 3,9 g Natriumhydrid (55%ige Mineralölsuspension) in 200 ml Dimethoxyäthan tropft man bei 0° C eine Lösung von 24 g Phosphonofluoressigsäureäthylester in 60 ml Dimethoxyäthan, rührt 1,5 Stunden, fügt eine Lösung von 16 g des vorstehend hergestellten Aldehyds in 50 ml Dimethoxyäthan zu und rührt 2 Stunden bei 0° C. Dann wird mit gesätt. Ammoniumchloridlösung versetzt, mit Äther extrahiert, mit Wasser gewaschen, mit Magnesiumsulfat getrocknet und im Vakuum eingeengt. Nach Chromatographie des Rückstands an Kieselgel mit Hexan/Äther (4+1) erhält man 17 g des $\alpha,\beta$-ungesättigten Esters.

Zur Hydrierung schüttelt man den Ester bei 25° C in 750 ml Essigester mit 1,6 g Palladium (10%ig auf Kohle) 6 Stunden unter einer Wasserstoffatmosphäre. Man filtriert und engt im Vakuum ein. Den Rückstand reinigt man durch Kugelrohrdestillation bei 0,2 Torr und 140° C. Dabei erhält man 11 g der Titelverbindung als farblose Flüssigkeit.
IR: 2950, 2913, 2858, 1735, 1460, 1373, 1250, 1100, 831/cm.

## Beispiel 2

(5E)-(16RS)-5-Fluor-16-methyl-18,18,19,19-tetradehydro-6a-carba-prostaglandin-$I_2$

In Analogie zu Beispiel 1 erhält man aus 340 mg des nach Beispiel 1 hergestellten (5E)-(16RS)-2-Descarboxy-5-fluor-2-hydroxymethyl-16-methyl-18,18,19,19-tetradehydro-6a-carba-prostaglandin-$I_2$-11,15-bis-(tetrahydropyranyläther) 195 mg der Titelverbindung als farbloses Öl.
IR: 3600, 3410 (breit), 2922, 1720, 1601, 970/cm.

## Beispiel 3

(5Z)-(16RS)-16,20-Dimethyl-5-fluor-18,18,19,19-tetradehydro-6a-carba-prostaglandin-$I_2$

In Analogie zu Beispiel 1 erhält man aus 840 mg (5E,Z)-(16RS)-2-Descarboxy-5-fluor-16,20-dimethyl-2-(dimethyl-tert.-butylsilyloxymethyl)-18,18,19,19-tetradehydro-6a-carba-prostaglandin-$I_2$-11,15-bis-(tetrahydropyranyläther) und Tetrabutylammoniumfluorid 290 mg (5Z)-(16RS)-2-Descarboxy-16,20-dimethyl-5-fluor-2-hydroxymethyl-18,18,19,19-tetradehydro-6a-carba-prostaglandin-$I_2$-11,15-bis-(tetrahydropryanyläther). Nach Oxidation und Schutzgruppenabspaltung erhält man 170 mg der Titelverbindung als farbloses Öl.
IR: 3600, 3410 (breit), 2921, 2868, 1719, 1601, 970/cm.

Das Ausgangsmaterial für die obige Titelverbindung wird wie folgt hergestellt:

3a)

(5E,Z)-(16RS)-2-Descarboxy-5-fluor-16,20-dimethyl-2-(dimethyl-tert.-butylsilyloxymethyl)-18,18,19,19-tetradehydro-6a-carba-prostaglandin-$I_2$-11,15-bis-(tetrahydropyranyläther)

In Analogie zu Beispiel 1 a erhält man aus 1,8 g (IR,5S,6R,7R)-7-(Tetrahydropyran-2-yolxy)-6-[(E)-(3S,4RS)-4-methyl-3-(tetrahydropyran-2-yolxy)-non-1-en-6-inyl]-bicyclo[3.3.0]-octan-3-on 0,7 g der Titelverbindung als farbloses Öl.
IR: 2947, 2870, 1451, 970, 835/cm.

## Beispiel 4

(5Z)-5-Fluor-20-methyl-18,18,19,19-tetradehydro-16,16-trimethylen-6a-carba-prostaglandin-$I_2$

In Analogie zu Beispiel 1 erhält man aus 920 mg (5E,Z)-2-Descarboxy-2-(dimethyl-tert.-butylsilyloxymethyl)-5-fluor-20-methyl-18,18, 19,19-tetradehydro-16,16-trimethylen-6a-carba-prostaglandin-$I_2$-11,15-bis-(tetrahydropyranyläther) und Tetrabutylammoniumfluorid 320 mg (5Z)-2-Descarboxy-5-fluor-2-hydroxymethyl-20-methyl-18,18, 19,19-tetradehydro-16,16-trimethylen-6a-carba-prostaglandin-$I_2$-11,15-bis-(tetrahydropyranyläther). Nach Oxidation und Schutzgruppenabspaltung erhält man 220 mg der Titelverbindung als farbloses Öl.
IR: 3610, 3400 (breit), 2920, 2870, 1720, 1601, 971/cm.

Das Ausgangsmaterial für die obige Titelverbindung wird wie folgt hergestellt:

4 a)

(5E,Z)-2-Descarboxy-2-(dimethyl-tert.-butylsilyloxymethyl)-5-fluor-20-methyl-18,18,19,19-tetradehydro-16,16-trimethylen-6a-carba-prostaglandin-$I_2$-11,15-bis-(tetrahydropyranyläther)

In Analogie zu Beispiel 1 a erhält man aus 1,4 g (1R,5S,6R,7R)-7-(Tetrahydropyran-2-yloxy)-6-[(E)-(3R)-3-(tetrahyropyran-2-yloxy)-4,4-trimethylen-non-1-en-6-inyl]-bicyclo[3.3.0]octan-3-on 0,55 g der Titelverbindung als farbloses Öl.
IR: 2950, 2868, 1451, 971, 835/cm.

Beispiel 5

(5Z)-16,16-Dimethyl-5-fluor-18,18,19,19-tetradehydro-6a-carba-prostaglandin-$I_2$

In Analogie zu Beispiel 1 erhält man aus 0,9 g (5E,Z)-2-Descarboxy-16,16-dimethyl-2-(dimethyl-tert.-butylsilyloxymethyl)-5-fluor-18,18,19,19-tetradehydro-6a-carba-prostaglandin-$I_2$-11,15-bis-(tetrahydropyranyläther) und Tetrabutylammoniumfluorid 310 mg (5Z)-2-Descarboxy-16,16-dimethyl-5-flour-2-hydroxymethyl-18,18, 19,19-tetradehydro-6a-carba-prostaglandin-$I_2$-11,15-bis-(tetrahydropyranyläther). Nach Oxidation und Schutzgruppenabspaltung erhält man 195 mg der Titelverbindung als farbloses Öl.
IR: 3600, 3420 (breit), 2922, 2868, 1718, 1600, 972/cm.

Das Ausgangsmaterial für die obige Titelverbindung wird wie folgt hergestellt:

5a)

(5E,Z)-2-Descarboxy-16,16-dimethyl-2-(dimethyl-tert.-butylsilyloxymethyl)-5-fluor-18,18,19,19-tetradehydro-6a-carba-prostaglandin-$I_2$-11,15-bis-(tetrahydropyranyläther)

In Analogie zu Beispiel 1 a erhält man aus 1,6 g (1R,5S,6R,7R)-7-(Tetrahydropyran-2-yloxy)-6-[(E)-(3R)-4,4-dimethyl-3-(tetrahydropyran-2-yloxy)-oct-1-en-6-inyl]-bicyclo[3.3.0]octan-3-on 0,7 g der Titelverbindung als farbloses Öl.
IR: 2950, 2870, 1450, 972, 835/cm.

Beispiel 6

(5Z)-5-Fluor-18,18,19,19-tetradehydro-16,16,20-trimethyl-6a-carba-prostaglandin-$I_2$

In Analogie zu Beispiel 1 erhält man aus 1,1 g (5E,Z)-2-Descarboxy-2-(dimethyl-tert.-butylsilyloxymethyl)-5-fluor-18,18,19,19-tetradehydro-16,16,20-trimethyl-6a-carba-prostaglandin-$I_2$-11,15-bis-(tetrahydropyranyläther) und Tetrabutylammoniumfluorid 380 mg (5Z)-2-Descarboxy-5-fluor-2-hydroxymethyl-18,18,19,19-tetradehydro-16,16,20-trimethyl-6a-carba-prostaglandin-$I_2$-11,15-bis-(tetrahydropyranyläther). Nach Oxidation und Schutzgruppenabspaltung erhält man 240 mg der Titelverbindung als farbloses Öl.
IR: 3610, 3400 (breit), 2925, 2870, 1718, 1602, 972/cm.

Das Ausgangsmaterial für die obige Titelverbindung wird wie folgt hergestellt:

6a)

(5E,Z)-2-Descarboxy-2-(dimethyl-tert.-butylsilyloxymethyl)-5-fluor-18,18,19,19-tetradehydro-16,16,20-trimethyl-6a-carba-prostaglandin-$I_2$-11,15-bis-(tetrahydropyranyläther)

In Analogie zu Beispiel 1 a erhält man aus 1,8 g (1R,5S,6R,7R)-7-(Tetrahydropyran-2-yloxy)-6-[(E)-(3R)-4,4-dimethyl-3-(tetrahydropyran-2-yl)-non-1-en-6-inyl]-bicyclo[3.3.0]octan-3-on 0,95 g der Titelverbindung als farbloses Öl.
IR: 2952, 2868, 1450, 972, 835/cm.

Beispiel 7

(5Z)-(16RS)-13,14-Didehydro-5-fluor-16-methyl-18,18,19,19-tetradehydro-6a-carba-prostaglandin-$I_2$

In Analogie zu Beispiel 1 erhält man aus 2 g (5E,Z)-2-descarboxy-13,14-didehydro-2-(dimethyl-tert.-butylsilylmethyl)-5-fluor-16-methyl-18,18,19,19-tetradehydro-6a-carba-prostaglandin-$I_2$-11,15-bis-(tetrahydropyranyläther) und Tetrabutylammoniumfluorid 0,79 g (5Z)-2-Descarboxy-13,14-didehydro-5-fluor-2-hydroxymethyl-16-methyl-18,18,19,19-tetradehydro-6a-carba-prostaglandin-$I_2$-11,15-bis-(tetrahydropyranyläther). Nach Oxidation und Schutzgruppenabspaltung erhält man 400 mg der Titelverbindung als farbloses Öl.
IR: 3600, 3405 (breit), 2928, 2872, 2221, 1720, 1600/cm.

Das Ausgangsmaterial für die obige Titelverbindung wird wie folgt hergestellt:

7 a)

(5E,Z)-2-Descarboxy-13,14-dedihydroxy-2-(dimethyl-tert.-butylsilyloxymethyl)-5-fluor-16-methyl-18,18,19,19-tetradehydro-6a-carba-prostaglandin-$I_2$-11,15-bis-(tetrahydropyranyläther)

In Analogie zu Beispiel 1 a erhält man aus 2,0 g (1R,5S,6S,7R)-7-(Tetrahydropyran-2-yloxy)-6-[-(3S,4RS)-4-methyl-3-(tetrahydropyran-2-yloxy)-octa-1,6-diinyl]-bicyclo[3.3.0]octan-3-on 1,05 g der Titelverbindung als farbloses Öl.
IR: 2956, 2875, 2221, 1458/cm.

Beispiel 8

(5Z)-(16RS)-13,14-Didehydro-16,20-dimethyl-5-fluor-18,18,19,19-tetradehydro-6a-carba-prostaglandin-$I_2$

In Analogie zu Beispiel 1 erhält man aus 1,90 g (5E,Z)-2-Descarboxy-13,14-didehydro-16,20-dimethyl-2-(dimethyl-tert.--butylsilyloxymethyl)-5-fluor-18,18,19,19-tetradehydro-6a-carba-prostaglandin-$I_2$-11,15-bis-(tetrahydropyranyläther) und Tetrabutylammoniumfluorid 0,71 g (5Z)-2-Descarboxy-13,14-didehydro-16,20-dimethyl-5-fluor-2-hydroxymethyl-18,18,19,19-tetradehydro-6a-carba-prostaglandin-$I_2$-11,15-bis-(tetrahydropyranyläther). Nach Oxidation un Schutzgruppenabspaltung erhält man 320 mg der Titelverbindung als farbloses Öl.
IR: 3600, 3405 (breit), 2926, 2870, 2221, 1715, 1110, 1025/cm.

8 a)

(5E,Z)-2-Descarboxy-13,14-didehydro-16,20-dimethyl-2-(dimethyl-tert.-butylsilyloxymethyl)-5-fluor-18,18,19,19-tetradehydro-6a-carba-prostaglandin-$I_2$-11,15-bis-(tetrahydropyranyläther)

In Analogie zu Beispiel 1 a erhält man aus 2,80 g (1R,5S,6S,7R)-7-(Tetrahydropyran-2-yloxy)-6-[-(3S,4RS)-4-methyl-3-(tetrahydropyran-2-yloxy)-nona-1,6-diinyl]-bicyclo[3.3.0]octan-3-on 1,39 g der Titelverbindung als farbloses Öl.
IR: 2951, 2872, 2220, 1460/cm.

Beispiel 9

(5Z)-13,14-Didehydro-5-fluor-20-methyl-18,18,19,19-tetradehydro-16,16-trimethylen-6a-carba-prostaglandin-$I_2$

In Analogie zu Beispiel 1 erhält man aus 2,0 g (5E,Z)-2-Descarboxy-13,14-didehydro-2-(dimethyl-tert.-butylsilyloxymethyl)-5-fluor-20-methyl-18,18,19,19-tetradehydro-16,16-trimethylen-6a-carba-prostaglandin-$I_2$-11,15-bis-(tetrahydropyranyläther) und Tetrabutylammoniumfluorid 0,75 g (5Z)-2-Descarboxy-13,14-didehydro-5-fluor-2-hydroxymethyl-20-methyl-18,18,19,19-tetradehydro-16,16-trimethylen-6a-cabra-prostaglandin-$I_2$-11,15-bis-(tetrahydropyranyläther). Nach Oxidation und Schutzgruppenabspaltung erhält man 328 mg der Titelverbindung als farbloses Öl.
IR: 3600, 3420 (breit), 2930, 2871, 2220, 1718, 1115, 1020/cm.

9 a)

(5E,Z)-2-Descarboxy-13,14-didehydro-2-(dimethyl-tert.-butylsilyloxymethyl)-5-fluor-20-methyl-18,18,19,19-tetradehydro-16,16-trimethylen-6a-carba-prostaglandin-$I_2$-11,15-bis-(tetrahydropyranyläther)

In Analogie zu Beispiel 1 a erhält man aus 2,5 g (1R,5S,6S,7R)-7-(Tetrahydropyran-2-yloxy)-6-[(3S)-3-(tetrahydropyran-2-yloxy)-4,4-trimethylen-nona-1,6-diinyl]-bicyclo[3.3.0]octan-3-on 1,21 g der Titelverbindung als farbloses Öl.
IR: 2952, 2868, 2218, 1458/cm.

Beispiel 10

(5Z)-13,14-Didehydro-5-fluor-18,18,19,19-tetradehydro-16,16,20-trimethyl-6a-carba-prostaglandin-$I_2$

In Analogie zu Beispiel 1 erhält man aus 2 g (5E,Z)-2-Descarboxy-13,14-didehydro-2-(dimethyl-tert.-butylsilyloxymethyl)-5-fluor-18,18,19,19-tetradehydro-16,16,20-trimethyl-6a-carba-prostaglandin-$I_2$-bis-(tetrahydropyranyläther) und Tetrabutylammoniumfluorid 0,71 g (5Z)-2-Descarboxy-13,14-didehydro-5-fluor-2-hydroxymethyl-18,18,19,19-tetradehydro-16,16,20-trimethyl-6a-carba-prostaglandin-$I_2$-11,15-bis-(tetrahydropyranyläther). Nach Oxidation und Schutzgruppenabspaltung erhält man 380 mg der Titelverbindung als farbloses Öl.
IR: 3600, 3410 (breit), 2938, 2870, 2225, 1713, 1120, 1018/cm.

10 a)

(5E,Z)-2-Descarboxy-13,14-didehydro-2-(dimethyl-tert.-butylsilyloxymethyl)-5-fluor-18,18,19,19-tetradehydro-16,16,20-trimethyl-6a-carba-prostaglandin-$I_2$-11,15-bis-(tetrahydropyranyläther)

In Analogie zu Beispiel 1 a erhält man aus 4,5 g (1R,5S,6S,7R)-7-(Tetrahydropyran-2-yloxy)-6-[(3S)-4,4-dimethyl-3-(tetrahydropyran-2-yloxy)-nona-1,6-diinyl]-bicyclo[3.3.0]octan-3-on 2,45 g der Titelverbindung als farbloses Öl.
IR: 2951, 2873, 2220, 1451/cm.

Beispiel 11

(5Z)-(16RS)-5-Fluor-16-methyl-18,18,19,19-tetradehydro-6a-carba-prostaglandin-$I_2$-methylester

Zu einer Lösung von 150 mg (5Z)-(16RS)-5-Fluor-16-methyl-18,18, 19,19-tetradehydro-6a-carba-prostaglandin-$I_2$ in 10 ml Dichlormethan tropft man bei 0° C eine ätherische Lösung von Diazomethan bis zur bleibenden Gelbfärbung. Nach weiteren 5 Minuten engt man im Vakuum ein und reinigt den Rückstand

durch Chromatographie an Kieselgel. Mit Toluol/2%Isopropylalkohol eluiert man 130 mg der Titelverbindung als farbloses Öl.
IR: 3600, 2937, 2866, 1450, 1435, 1025, 976/cm.

## Patentansprüche

**1.** 5-Fluorcarbacyclinderivate der Formel I

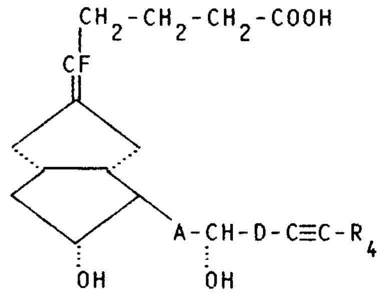

(I),

worin

A eine trans-CH = CH- oder -C≡C-Gruppe,
D die Gruppe

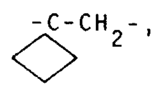

wenn $R_4$ Ethyl bedeutet, oder die Reste $-CH(CH_3)-CH_2-$ oder $-C(CH_3)_2-CH_2-$,
$R_4$ Methyl oder Ethyl bedeuten und deren Salze mit physiologisch verträglichen Basen.

**2.** (5Z)-(16RS)-5-Fluor-16-methyl-18,18,19,19-tetrahydro-6a-carbaprostaglandin-$I_2$.

**3.** Verfahren zur Herstellung der 5-Fluorcarbacyclin-Derivate der allgemeinen Formel I, dadurch gekennzeichnet, daß man in an sich bekannter Weise eine Verbindung der allgemeinen Formel II

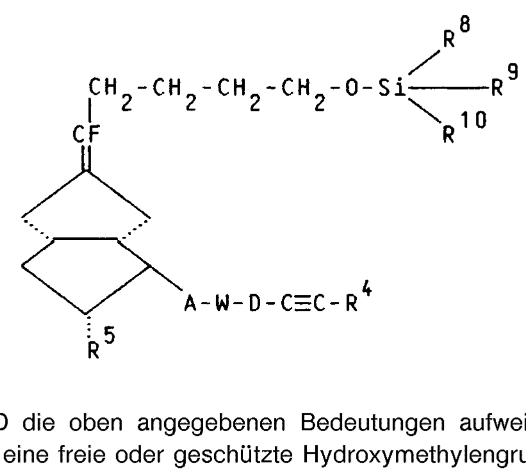

(II),

worin $R^4$, A und D die oben angegebenen Bedeutungen aufweisen, $R^5$ eine freie oder geschützte Hydroxygruppe, W eine freie oder geschützte Hydroxymethylengruppe und $R^8$, $R^9$ und $R^{10}$ gleich oder verschieden sein können und eine geradkettige oder verzweigtkettige Alkylgruppe mit 1-10 C-Atomen oder eine Arylgruppe bedeuten, gegebenenfalls nach Schutz anwesender freier Hydroxygruppen mit einem Reagenz, das zur Spaltung von Silylgruppen geeignet ist, umsetzt und gegebenenfalls anschließend in beliebiger Reihenfolge Isomere trennt und/oder geschützte Hydroxygruppen freisetzt und die 1-Hydroxygruppe zur 1-Carboxylgruppe oxidiert und geschützte Hydroxygruppen freisetzt und/oder mit einer physiologisch verträglichen Base in ein Salz überführt.

**4.** Arzneimittel, bestehend aus einer oder mehreren Verbindungen gemäß Anspruch 1 und üblichen Hilfs- und Trägerstoffen.

**Claims**

**1.** 5-Fluorocarbacyclin derivatives of the formula I

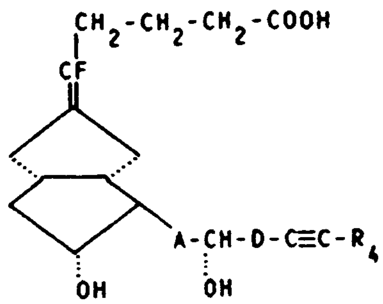

(I)

wherein

A represents a trans-CH = CH- or -C≡C- group,

D represents the group

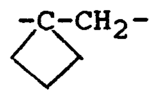

when $R_4$ represents ethyl, or the radicals $-CH(CH_3)-CH_2-$ or $C(CH_3)_2-CH_2-$,

$R_4$ represents methyl or ethyl, and salts thereof with physiologically tolerable bases.

**2.** (5Z)-(16RS)-5-fluoro-16-methyl-18,18,19,19-tetrahydro-6a-carbaprostaglandin-$I_2$.

**3.** Process for the manufacture of the 5-fluorocarbacyclin derivatives of the general formula I, characterised in that, in a manner known per se, a compound of the general formula II

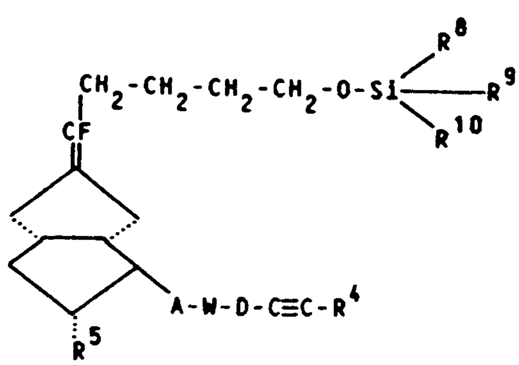

(II),

wherein $R^4$, A and D have the meanings given above, $R^5$ may be a free or protected hydroxy group, W may be a free or protected hydroxymethylene group and $R^8$, $R^9$ and $R^{10}$ may the same or different and represent a straight-chain or branched-chain alkyl group having from 1 to 10 C-atoms or an aryl group, is reacted, optionally after protection of free hydroxy groups present, with a reagent that is suitable for cleaving silyl groups and, optionally, subsequently, in any desired sequence, isomers are separated and/or protected hydroxy groups are freed and the 1-hydroxy group is oxidised to the 1-carboxy group and protected hydroxy groups are freed and/or converted into a salt with a physiologically tolerable base.

**4.** Medicament, consisting of one or more compounds according to claim 1 and customary auxiliaries and carriers.

**Revendications**

**1.** Fluoro-5 carbacyclines répondant à la formule I :

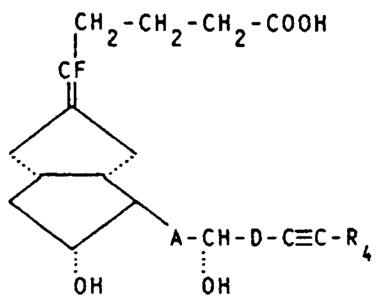

(I)

dans laquelle

A représente un radical -CH = CH- trans ou un radical -C≡C-,

D représente un radical

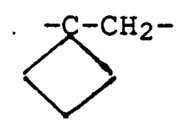

dans le cas où $R_4$ est un éthyle, ou représente un radical $-CH(CH_3)-(CH_2)-$ ou $-C(CH_3)_2-CH_2-$, et

$R^4$ représente un radical méthyle ou éthyle, ainsi que les sels qu'elles forment avec des bases acceptables du point de vue physiologique.

**2.** Fluoro-5 méthyl-16 tétradéhydro-18,18,19,19 carba-6a prostaglandine $I_2$ (5Z)-(16RS).

**3.** Procédé pour préparer les fluoro-5 carbacyclines de formule générale I, procédé caractérisé en ce que, en opérant de manière connue, on fait réagir un composé répondant à la formule générale II :

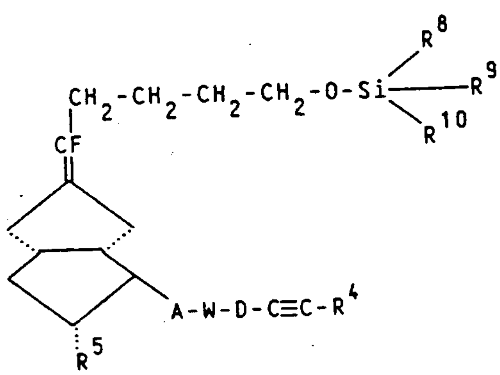

(II)

dans laquelle $R^4$, A et D ont les significations qui leur ont été données ci-dessus, $R^5$ représente un radical hydroxy libre ou protégé, W représente un radical hydroxyméthylène libre ou protégé, et $R^8$, $R^9$ et $R^{10}$ représentent chacun, indépendamment l'un de l'autre, un radical alkyle en $C_1$-$C_{10}$, linéaire ou ramifié, ou un radical aryle, avec un réactif convenant pour la coupure de radicaux silyles, après quoi,

le cas échéant, en opérant dans l'ordre que l'on veut, on sépare des isomères et/ou on libère des radicaux hydroxy protégés et on oxyde le radical hydroxy en 1 pour obtenir un radical carboxy à carbone C-1 et on libère des radicaux hydroxy protégés, ou on transforme le composé en un sel avec une base acceptable du point de vue physiologique.

**4.** Médicaments constitués d'un ou de plusieurs composés selon la revendication 1 et d'adjuvants et excipients usuels.